# FASCICULE DE BREVET EUROPEEN

(11) **EP 0 968 717 B1**
(45) Date de publication et mention de la délivrance du brevet: **07.06.2006**
(21) Numéro de dépôt: 99450012.2
(22) Date de dépôt: 02.06.1999
(51) Int. Cl.: A61K 31/565, A61K 31/57, A61P 5/00

(54) **Préparation sous forme galénique simple visant à limiter les effects induits durant la ménopause et postérieurement et traitement avec cette préparation**
Arzneimittel zur Verminderung der Effekte während oder nach der Menopause und Verfahren tur Behandlung under Verwendung derselben
Galenic formulatin for reducing effects induced during or after menopause and method of treatment using same

(30) Priorité: 02.06.1998 FR 9807051
(43) Date de publication de la demande: 05.01.2000
(73) Titulaire: Amistad Pharma SAS, 21000 Dijon (FR)
(72) Inventeur: Auzerie, Jack, 33560 Sainte-Eulalie (FR)
(74) Mandataire: Carpmaels & Ransford

(56) Documents cités:
- EP-A- 0 285 563
- WO-A-96/10991
- DE-A- 4 019 670
- DE-A- 19 526 864
- J. SLOWINSKA-SRZEDNICKA ET AL: "Transdermal 17-beta-estradiol combined with oral progestogen increases plasma levels of insulin-like growth factor-I in postmenopausal women" JOURNAL OF ENDOCRINOL. INVEST., vol. 15, 1992, pages 533-538, XP002094051
- C. GUAL ET AL: "Regulatory effects of steroids on the pituitary response to LH-RH" JOURNAL OF STEROID BIOCHEMISTRY, vol. 6, 1975, pages 1067-1074, XP002094052

## Description

La présente invention concerne une préparation sous forme galénique simple visant à limiter les effets induits durant la ménopause et postérieurement. L'invention couvre aussi le traitement préconisé avec cette préparation.

Il existe des besoins en préparation dans le domaine du traitement hormonal substitutif.

En effet, à la suite de la ménopause, il a été constaté que la prise d'hormones permettait de pallier ou tout au moins de réduire les risques d'ostéoporose, les accidents cardio-vasculaires ou l'apparition de la maladie d'Alzheimer.

Les applications concernent notamment les ménopauses anciennes, au-delà de 3 ans et d'après les enquêtes connues à ce jour, la très grande majorité des femmes souhaite un traitement qui ne nécessite pas le retour des règles. En effet, pour une moyenne d'âge de 54 ans et après une interruption des hémorragies cycliques de plusieurs années, le traitement sans règles est un impératif.

On connaît un traitement estro-progestatif, mené uniquement à titre de campagne de tests, qui consiste à combiner de l'estradiol percutané et de la progestérone micronisée par voie orale, ceci sur des durées d'administration de 25 jours par mois avec une interruption de 5 jours à 7 jours par mois.

D'autres traitements connus et actuellement administrés consistent à prendre de nombreux produits combinés, à des heures données de la journée sur des périodes séquencées dont les cycles se superposent si bien que ces contraintes conduisent souvent à l'abandon du traitement, surtout chez les sujets les plus âgées, si bien que seulement 10 à 20% des femmes ménopausées sont traitées et pour la plupart d'entre elles, dans les trois années qui suivent. Le pourcentage de femmes traitées est encore plus faible au-delà.

De plus, les traitements sont bien souvent préconisés pour soigner des troubles symptomatiques et non préconisés en vue d'un traitement à long terme pour améliorer la qualité du vieillissement.

Les traitements connus demandent aussi de 9 à 12 mois pour supprimer tout saignement et/ou métrorragie très gênant, ce qui est long et qui est aussi une des causes d'abandon prématuré du traitement.

On a noté que, en fonction des patientes, certaines posologies recommandées, 2 mg d'oestrogènes par voie buccale ou 50 µg transdermique, ne sont pas tolérées et ceci en fonction du temps. Les professionnels de la santé ne peuvent suivre en continu les patientes or:
- trop de rigidité dans le traitement amène la patiente à supprimer le traitement pour troubles jugés intolérables, et
- un sous-dosage entraîne un résultat sub-optimal, si bien que, l'objectif n'étant pas atteint, la patiente a une bonne raison d'abandonner le traitement hormonal substitutif.

Il faut donc que la patiente puisse moduler la posologie et que le prise s'effectue une fois par jour avec un produit seul et unique et par voie buccale qui est la plus simple et la plus rapide et, surtout, celle qui génère le moins de contraintes.

L'art antérieur décrit des préparations comprenant, en combinaison, du 17β oestradiol et de l'acétate de chlormadinone (voir WO 96/10991, EP 285 563 et DE 4019670).

La présente invention consiste à proposer une préparation sous forme galénique simple qui pallie les inconvénients précités et qui permet une modulation du traitement.

A cet effet, selon l'invention, la préparation visant à limiter les effets induits durant la ménopause et postérieurement, sans l'apparition de règles, caractérise en ce qu'elle comprend 2 mg de 17β oestradiol et 2 mg d'acétate de chlormadinone.

Le 17β oestradiol est sous forme micronisée.

La forme galénique simple retenue qui permet de résoudre le problème de l'adaptation du traitement et d'apporter la simplicité de la prise consiste en un comprimé.

Le comprimé est sécable pour permettre un demi-dosage.

L'invention concerne aussi utilisation de 17β-oestradiol et d'acétate de chlormadinone dans la fabrication d'un produit thérapeutique qui contient des 25 doses quotidiennes séquentielles combinées pour traiter pendant un mois les effets induits durant la ménopause et postérieurement chez une patiente, le traitement ne faisant pas apparaître de règles, et dans laquelle chaque dose quotidienne combinée comprend 2 mg de 17β-oestradiol et 2 mg d'acétate de chlormadinone.

L'invention concerne aussi utilisation de 1 mg 17β-oestradiol et 1 mg d'acétate de chlormadinone dans la fabrication d'un produit thérapeutique pour traiter les effets induits durant la ménopause et postérieurement chez une patiente, le traitement ne faisant pas apparaître de règles.

L'invention concerne aussi utilisation de 17β-oestradiol et d'acétate de chlormadinone dans la fabrication d'un produit thérapeutique qui contient des 25 doses quotidiennes séquentielles combinées pour traiter pendant un mois les effets induits durant la ménopause et postérieurement chez une patiente, le traitement ne faisant pas apparaître de règles, et dans laquelle chaque dose quotidienne combinée se présentent sous la forme de comprimés sécables et qu'une demi-dose quotidienne combinée comprend 1 mg de 17β-oestradiol et 1 mg d'acétate de chlormadinone.

L'invention concerne aussi le traitement correspondant, sans apparition de règles, des femmes ménopausées, avec cette préparation, traitement qui consiste à administrer à une patiente, un demi à un comprimé par jour, pendant 25 jours par mois avec une interruption de 5 à 7 jours.

Selon l'invention, la tolérance des progestatifs pose des problèmes, soit par leur nature, soit par leur posologie.

Certains progestatifs par leur nature, norstéroïdes, posent des problèmes de tolérance glucidique, de virilisation et même de participation sur la cancérogenèse. Les effets induits sont également problématiques dans certains cas, prise de poids, somnolence ou troubles digestifs.

Les doses actuelles sont trop élevées pour atteindre le but thérapeutique de la présente invention car les progestatifs n'ont pour vocation que de bloquer l'hyperplasie utérine muqueuse.

Il faut donc réduire ces doses surtout dans le cas de schémas continus.

La présente préparation consiste à combiner du 17β oestradiol, de préférence sous forme micronisée et de l'acétate de chlormadinone à raison de 2 mg de chaque. Un traitement sur un mois avec une interruption de 5 à 7 jours est adapté.

La forme galénique simple retenue est un comprimé unique dosé pour un jour et sécable pour permettre de diminuer de moitié la dose administrée. Aussi, la patiente est à même de diminuer la dose prescrite en fonction de la tolérance sans pour autant arrêter le traitement et sans complication dans la prise de ce traitement puisqu'il suffit de séparer le comprimé en deux et de n'en prendre qu'une moitié.

De plus, il est possible de vérifier immédiatement que la dose prise est une dose complète ou moitié, ce qui n'est pas le cas dans les posologies qui incluent des traitements percutanés ou par voie orale avec des comprimés non sécables.

Les boîtes de présentation sont de type à 25 comprimés, ce qui assure le traitement pour un mois.

Cette présentation très simple est d'un usage très aisé, y compris pour les personnes âgées.

Le traitement débute au 1er du mois et se termine le 25 du même mois.

Les produits inclus dans cette composition sont d'un coût tout à fait compatible avec un traitement à long terme. Le prix de revient est inférieur à celui des autres produits. De même, les posologies sont diminuées en quantité par rapport aux posologies existantes.

On note également que le problème de la prise unique journalière est résolu, ce qui évite d'avoir à gérer un pilulier.

## Revendications

1. Préparation visant à limiter les effets induits durant la ménopause et postérieurement, sans l'apparition de règles, **caractérisée en ce qu'**elle comprend 2 mg de 17β oestradiol et 2 mg d'acétate de chlormadinone

2. Préparation suivant la revendication 1, **caractérisée en ce qu'**elle peut être administrée par voie orale.

3. Préparation suivant l'une quelconque des revendications 1 ou 2, **caractérisée en ce qu'**elle se présente sous la forme d'un comprimé.

4. Préparation suivant la revendication 3, **caractérisée en ce que** le comprimé est sécable pour permettre un demi-dosage.

5. Préparation suivant l'une quelconque des revendications 1 à 4, destinée à être utiliser dans le traitement des effets induits durant la ménopause et postérieurement, sans l'apparition de règles.

6. Utilisation de 17β-oestradiol et d'acétate de chlormadinone dans la fabrication d'un produit thérapeutique qui contient des 25 doses quotidiennes séquentielles combinées pour traiter pendant un mois les effets induits durant la ménopause et postérieurement chez une patiente, le traitement ne faisant pas apparaître de règles, et dans laquelle chaque dose quotidienne combinée comprend 2 mg de 17β-oestradiol et 2 mg d'acétate de chlormadinone.

7. Utilisation de 1 mg 17β-oestiadiol et 1 mg d'acétate de chlormadinone dans la fabrication d'un produit thérapeutique pour traiter les effets induits durant la ménopause et postérieurement chez une patiente, le traitement ne faisant pas apparaître de règles.

8. Utilisation de 17β-oestradiol et d'acétate de chlormadinone dans la fabrication d'un produit thérapeutique qui contient des 25 doses quotidiennes séquentielles combinées pour traiter pendant un mois les effets induits durant la ménopause et postérieurement chez une patiente, le traitement ne faisant pas apparaître de règles, et dans laquelle chaque dose quotidienne combinée se présentent sous la forme de comprimés sécables et qu'une demi-dose quotidienne combinée comprend 1 mg de 17β-oestradiol et 1 mg d'acétate de chlormadinone.

9. Trousse destinée à être utilisée pendant un mois pour limiter les effets induits durant la ménopause et postérieurement, sans l'apparition de règles, **caractérisée en ce qu'**elle consiste en 25 doses quotidiennes séquentielles, chaque dose quotidienne comprenant en combinaison 2 mg de 17β-estradiol et 2 mg d'acétate de chlormadinone.

10. Trousse suivant la revendication 9, dans laquelle chaque dose quotidienne peut être administrée par voie orale.

11. Trousse suivant la revendication 9 ou 10, dans laquelle la dose quotidienne se présente sous la forme d'un comprimé.

12. Trousse suivant la revendication 11, dans laquelle chaque dose quotidienne combinée est sécable pour permettre un demi-dosage.

13. L'utilisation d'une trousse suivant l'une quelconque des revendications 9 à 12, pour la fabrication d'un produit thérapeutique qui contient des doses quotidiennes combinées pour traiter pendant un mois les effets induits durant la ménopause et postérieurement chez une patiente, le traitement ne faisant pas apparaître de règles.

## Claims

1. Preparation directed towards limiting the effects induced during the menopause and subsequently, without the onset of periods, **characterized in that** it comprises 2 mg of 17β-oestradiol and 2 mg of chlormadinone acetate.

2. Preparation according to Claim 1, **characterized in that** it may be administered orally.

3. Preparation according to either of Claims 1 and 2, **characterized in that** it is in the form of a tablet.

4. Preparation according to Claim 3, **characterized in that** the tablet is splittable to allow a half-dosage.

5. Preparation according to any one of Claims 1 to 4, for use in the treatment of effects induced during the menopause and subsequently, without the onset of periods.

6. Use of 17β-oestradiol and of chlormadinone acetate in the manufacture of a therapeutic product containing 25 combined sequential daily doses to treat for one month the effects induced during the menopause and subsequently in a patient, the treatment not causing the onset of periods, and in which each combined daily dose comprises 2 mg of 17β-oestradiol and 2 mg of chlormadinone acetate.

7. Use of 1 mg of 17β-oestradiol and 1 mg of chlormadinone acetate in the manufacture of a therapeutic product for treating the effects induced during the menopause and subsequently in a patient, the treatment not causing the onset of periods.

8. Use of 17β-oestradiol and of chlormadinone acetate in the manufacture of a therapeutic product containing 25 combined sequential daily doses to treat for one month the effects induced during the menopause and subsequently in a patient, the treatment not causing the onset of periods, and in which each combined daily dose is in the form of scored tablets and in that a combined daily half-dose comprises 1 mg of 17β-oestradiol and 1 mg of chlormadinone acetate.

9. Kit intended to be used for one month to limit the effects induced during the menopause and subsequently, without the onset of periods, **characterized in that** it consists of 25 sequential daily doses, each daily dose comprising in combination 2 mg of 17β-oestradiol and 2 mg of chlormadinone acetate.

10. Kit according to Claim 9, in which each daily dose may be administered orally.

11. Kit according to Claim 9 or 10, in which the daily dose is in the form of a tablet.

12. Kit according to Claim 11, in which each combined daily dose is scored to allow the administration of a half-dose.

13. Use of a kit according to any one of Claims 9 to 12, for the manufacture of a therapeutic product that contains combined daily doses for treating for one month the effects induced during the menopause and subsequently in a patient, the treatment not causing the onset of periods.

## Patentansprüche

1. Zubereitung zur Begrenzung der während und nach der Menopause aufgetretenen Auswirkungen, ohne dass die Regel auftritt, **dadurch gekennzeichnet, dass** sie 2 mg 17β-Östradiol und 2 mg Chlormadinonacetat enthält.

2. Zubereitung nach Anspruch 1, **dadurch gekennzeichnet, dass** sie oral verabreicht wird.

3. Zubereitung nach einem der Ansprüche 1 oder 2, **dadurch gekennzeichnet, dass** sie in Form einer Tablette vorliegt.

4. Zubereitung nach Anspruch 3, **dadurch gekennzeichnet, dass** die Tablette teilbar ist, um eine Halbdosierung zu ermöglichen.

5. Zubereitung nach einem der Ansprüche 1 bis 4 zur Verwendung bei der Behandlung der während und nach der Menopause aufgetretenen Auswirkungen, ohne dass die Regel auftritt.

6. Verwendung von 17β-Östradiol und Chlormadinonacetat zur Herstellung eines therapeutischen Erzeugnisses, das 25 kombinierte aufeinanderfolgende tägliche Dosen zur Behandlung über einen Monat der während und nach der Menopause bei einer Patientin aufgetretenen Auswirkungen enthält, wobei die Behandlung keine Regel auftreten läßt und wobei jede kombinierte tägliche Dosis 2 mg 17β-Östradiol und 2 mg Chlormadinonacetat enthält.

7. Verwendung von 1 mg 17β-Östradiol und 1 mg Chlormadinonacetat zur Herstellung eines therapeutischen Erzeugnisses zur Behandlung von während und nach der Menopause aufgetretenen Auswirkungen bei einer Patientin, wobei die Behandlung keine Regel auftreten läßt.

8. Verwendung von 17β-Östradiol und Chlormadinonacetat zur Herstellung eines therapeutischen Erzeugnisses, das 25 kombinierte aufeinanderfolgende tägliche Dosen zur Behandlung über einen Monat der während und nach der Menopause bei einer Patientin aufgetretenen Auswirkungen enthält, wobei die Behandlung keine Regel auftreten läßt und wobei jede kombinierte tägliche Dosis in Form von teilbaren Tabletten vorliegt und wobei eine tägliche kombinierte Halbdosis eine Kombination aus 2 mg 17β-Östradiol und 2 mg Chlormadinonacetat umfaßt.

9. Kit zur Verwendung während eines Monats zur Begrenzung der während und nach der Menopause aufgetretenen Auswirkungen, ohne dass die Regel auftritt, **dadurch gekennzeichnet, dass** es aus 25 aufeinanderfolgenden täglichen Dosen besteht, wobei jede tägliche Dosis in Kombination 2 mg 17β-Östradiol und 2 mg Chlormadinonacetat enthält.

10. Kit nach Anspruch 9, wobei jede tägliche Dosis oral verabreicht werden kann.

11. Kit nach Anspruch 9 oder 10, wobei die tägliche Dosis in Form einer Tablette vorliegt.

12. Kit nach Anspruch 11, wobei jede kombinierte tägliche Dosis teilbar ist, um eine Halbdosierung zu ermöglichen.

13. Verwendung eines Kits nach einem der Ansprüche 9 bis 12 zur Herstellung eines therapeutischen Mittels, das kombinierte tägliche Dosen enthält, um über einen Monat die während und nach der Menopause aufgetretenen Auswirkungen bei einer Patientin zu behandeln, wobei die Behandlung keine Regel auftreten läßt.
